# EUROPEAN PATENT SPECIFICATION

(11) **EP 2 744 467 B1**
(45) Date of publication and mention of the grant of the patent: **24.06.2020**
(21) Application number: 12791565.0
(22) Date of filing: 06.11.2012
(51) Int. Cl.: A61H 23/02, A61H 31/00

(54) **RESUSCITATION DEVICE**
WIEDERBELEBUNGSVORRICHTUNG
DISPOSITIF DE RÉANIMATION

(30) Priority: 08.11.2011 US 201161556855 P
(43) Date of publication of application: 25.06.2014
(73) Proprietor: Koninklijke Philips N.V., 5656 AG Eindhoven (NL)
(72) Inventor: WOERLEE, Pierre Hermanus, 5656 AE Eindhoven (NL); AELEN, Paul, 5656 AE Eindhoven (NL)
(74) Representative: Philips Intellectual Property & Standards
(86) International application number: PCT/IB2012/056180
(87) International publication number: WO 2013/068914

(56) References cited:
- WO-A2-99/63926
- WO-A2-99/65560
- US-A1- 2003 135 085
- US-A1- 2004 162 510

## Description

### FIELD OF THE INVENTION

The present invention relates to a resuscitation device.

### BACKGROUND OF THE INVENTION

Cardiac arrest (CA) is one of the main causes of death in the western world. After the heart has stopped pumping, death is unavoidable unless acute medical care is available. The resulting ischemia disturbs a wide range of cell processes; this eventually leads to cell death. It has been reported that the probability for survival after cardiac arrest decreases exponentially with time. After about 4 minutes the condition of heart and brain is deteriorated to such an extent that a minimum amount of perfusion (induced by for instance CPR or other means) is required to restore the cells and organs before further treatment (i.e. defibrillation) can be applied. Unfortunately the perfusion by CPR is very low even if done perfectly; hence further ischemia and cell damage during CPR is very likely.

A detrimental phenomenon which occurs after perfusion is restored (after Return of Spontaneous Circulation, ROSC) is so-called reperfusion damage. This is caused by providing oxygen and nutrients to cells which are in a bad condition due to a long (> 3-4 minutes) duration ischemia. From the 30-40% of the victims of CA that have ROSC roughly two third die due to this phenomenon. On average only 10% of all CA victims survive, many have an impaired neurological function. Hence there is a very high mortality rate after ROSC. There is a large need for treatment that reduces reperfusion damage and increase survival with good neurological outcome.

Recently research has indicated that reperfusion damage can be reduced by applying mild hypothermia (i.e. rapid cooling to 33 °C). This method has received class I recommendation by ILCOR. However, hypothermia is difficult to apply rapidly during cardiac arrest.

In the present description, a technique is discussed that can be used to increase both micro perfusion during CPR and reduce reperfusion damage after ROSC resulting in an improved outcome of cardiac arrest treatment. The proposed device enhances shear stress related emission of cardio- and neuro-protective endogenous substances from the endothelial lining of the (large) vessels (aorta, venae cava ...). Application of the technique before, during or after a period of reduced or negligible perfusion may have a positive influence on the degree of reperfusion damage and hence on CPR outcome. It is well known that the endothelial lining of the vessels emit substances when triggered by shear stress, these in turn cause local vasodilatation by relaxing the vascular smooth muscle, influence the inflammatory system in a positive way and reduce blood clothing and adhesion of lymphocytes on the vessel wall.

For CPR, vasodilation of the arterioles and capillaries in the heart and brain is very important, among others it causes better perfusion of the heart and brain (by reduced flow resistance) during CPR and therefore reduces ischemia. The vasodilation is mainly caused by NO emission due to the conversion of L-arginine by three different nitric oxide synthases enzymes (NOS). Other substances emitted due to shear stress (like prostacyclin, thromboxane) are claimed to have a beneficial influence on platelet aggregation. In the coronary vessels endothelial-dependent hyperpolarizing factor is released with subsequent significant coronary vasodilation. Tissue plasminogen activator is also released, it has a function in the dissolution of blood cloths. Of special interest is reduction of clothing in the micro-circulation in the brain and heart. Hence high shear stress on the endothelial lining of large vessels in the proper frequency range is desirable during CPR.

Due to the low-flow during CPR there is a very low shear stress and hence reduced emission of these endogenous substances. There is also a lack of blood oxygenation, which further slows down the NOS synthase pathway. This reduced emission of NO and other substances may cause vasoconstriction and enhances other detrimental processes (such as blood clothing, etc). Hence it is important to enhance the shear stress on the vessel lining both during and after CPR (due to pulsatile blood flow or other means).It is also important to induce sufficient oxygenated blood flow. Since CPR itself is not well suited to generate the required shear stress in the vessel lining other methods have been proposed.

It has been proposed to use whole body shaking in the head-to-toe direction. Typical shaking parameters are a frequency of a few Hz (optimum 3-6Hz) and accelerations around 1g. The so-called p-Gz CPR has shown superior results on (neurological) outcome in animal tests of ventricular fibrillation. This group has also shown both in vitro and in vivo that pGz shaking enhances NO and shear stress related endogenous substances emission. Adams has noted that pGz CPR alone is not sufficient and additional manual or mechanical CPR is required (US6878123). The pGz CPR method is not very practical for use during CPR and can also be dangerous for some patients. Therefore a simpler method to induce shear stress related emission of NO and related substances is highly required.

It is known that (ultra) sound (US) or vibrations can have therapeutic action on some physiological processes such as wound healing, bone growth and increase in perfusion. The frequency range where therapeutic action occurs is very wide from audio frequencies (kHz) to MHz's. Also there are claims that therapeutic US can reduce reperfusion damage after myocardial infarction and stroke. The mechanisms related to this reduced reperfusion damage are claimed to be similar to the ones described by Sackner for pGz CPR, i.e. US, sound or vibration can induce shear stress in the endothelial linings of large vessels and body cavities. There are many studies that demonstrated biological activity induced by US, both in vitro and in vivo. Patent document WO9965560 discloses a resuscitation system wherein electrodes are provided on the neck or chest of patient for electrically stimulating the diaphragm to provide inhalation and ventilation of the patient. The controller co-ordinates the operation of the chest compression unit with the electrostimulation unit, to enhance the effectiveness of the resuscitation system.

The inventor of the present invention has appreciated that an improved automatic resuscitation device is of benefit, that treatment to reduce reperfusion damage is needed already during resuscitation treatment and integration with high quality resuscitation devices/methods (e.g. automated CPR, hypothermia devices) is needed and has in consequence devised the present invention.

### SUMMARY OF THE INVENTION

It would be advantageous to achieve a device providing an increased likelihood of surviving cardiac arrest and at the same time ensuring that the device is easy to use for a non-skilled person.

In general, the invention preferably seeks to mitigate, alleviate or eliminate one or more of the above mentioned disadvantages singly or in any combination. In particular, it may be seen as an object of the present invention to provide a method that solves the above mentioned problems, or other problems, of the prior art.

To better address one or more of these concerns, according to the invention an apparatus for performing cardiopulmonary resuscitation is presented as defined in claim 1. The combination of a resuscitation device and a stimulation device is advantageous in that the person under treatment, i.e. a person having suffered an ischemia receives both a significant flow of oxygenated blood (needed to reduced ischemia) as well as cardio and neuro-protoctive substances (note that production of these substances also requires the presence of oxygenated blood) to reduce reperfusion damage. As mentioned elsewhere the combination of CPR and additional stimulation of the endothelial function in a patient is advantageous in that it lowers the risk for reperfusion damage, which in turn raises the chance of survival for patients suffering from ischemia.

The stimulation device is configured for stimulating the nitric oxide synthase pathway by transmitting vibrations when the stimulation device is in contact with the body of the patient. The vibrations are contemplated to travel through the body of the person to the intended areas, e.g. heart and other organs and body parts. The vibrations may be dampened through the body but this may be compensated for by altering the amplitude of the stimulation.

The stimulation device emits acoustic sound waves and/or ultrasound waves. This is contemplated to be advantageous in that sound is less prone to damping when traveling through the body, e.g. compared to light.

The vibrations may have preferably a frequency in the interval from 1 Hz to 10 MHz, such as 24-27 kHz. Specific intervals will be discussed throughout the present specification.
Advantageously the stimulation device may emit pulsed signals as this may in some instances be more effective than static fields. Also periods of rest, i.e. no stimulation, may allow the body to reinstate its normal operation.

Advantageously the stimulation device may emit power below 10 W/cm2, such as below 8 W/cm2, such as below 5 W/cm2, such as below 3 W/cm2, such as below 2, W/cm2, such as below 1 W/cm2. It is contemplated to be advantageous to emit signals at a relatively low power lever. Advantageously the stimulation device may emit power such that tissue damage does not occur. The emitted power level is such that local body heating is limited to below 1 degree Celsius and that clinical beneficial effects are significant. Typical power densities are around 1W/cm2 for ultra sound and acoustic sound waves but upper level values of 2-3W/cm2 may be used in some cases. The useable power density depends on the absorption coefficient of the radiation/vibration energy. It is contemplated to be advantageous to emit signals at a relatively low power level that still shows clinical benefit.

Advantageously the stimulation device may emit continuous or in a pulsed or burst mode with a specific duty cycle. It may be advantageous to have the apparatus delivering stimulation in a certain period of a first time following by a second period of time where no signal is emitted. Advantageously the duty cycle is between 1% and 90%, such as less than 50%.

Advantageously the stimulation device may be configured to stimulate the thorax and/or neck/head region of a patient in need thereof. The stimulation device is configured to target the stimulation to areas where the nitric oxide synthase pathways in a patient are present.

Advantageously the stimulation device may be combined with or integrated with a device for application of mild hypothermia. Both devices may be either used invasively or non-invasively, e.g. intravenously by integration in a catheter.

As an illustrative example, a method of operating an apparatus for performing cardiopulmonary resuscitation comprising a resuscitation device configured for autonomously performing cardiopulmonary resuscitation, a stimulation device configured for stimulating the nitric oxide synthase pathway in a patient in need thereof and a controller for controlling the resuscitation device and/or the stimulation device, may comprise the steps of operating the resuscitation device for delivering suitable resuscitation operations to a patient in need thereof, and operating the stimulation device so as to stimulate the endothelial function and nitric oxide synthase pathway in a patient in need thereof. It is very advantageous to combine the operation of a resuscitation device with the operation of a stimulation device and thereby increasing the effect due to the generation of relatively large flows of oxygenated blood by lowering the risk of perfusion damage. By coordinating the operation of such two devices the chance of saving lives of persons suffering from ischemia is raised.

Advantageously the method may further comprise the step of operating the stimulation device so as to stimulate the thorax and/or neck/head region of a patient in need thereof. It is advantageous to stimulate the area of the thorax and/or neck/head region of a patient as this is the areas where the largest perfusion damages may occur.

Instead of sound or ultra sound also mechanical vibrations with frequency below a few thousand hertz have been proposed to induce shear stress in the vessels and heart and even to promote dissolution of blood cloths in coronary arteries. There are also numerous papers and investigations on stimulation of these systems by (pulsed) electric or magnetic fields. Hence besides vigorous mechanical shaking there are more subtle, practical and more safe methods to promote these key endogenous processes during and after CPR. These transducers can be applied locally. It is however very important to combine these adjuncts with high quality CPR, preferably integrating these devices in an automated CPR device.

Advantageously the stimulation device further comprises a cooling device and the method comprises subjecting a patient in need thereof to hypothermia. The cooling of a patient could be advantageous as a lower temperature of the body of a person as this may help reduce complications when the heart has stopped.

Generally it is advantageous to provide a combination of mechanical and electro-magnetic adjuncts and an automated CPR device to improve CPR outcome. It is currently preferred that these adjuncts are based on sound, ultra sound and/or mechanical vibration, but other adjuncts may be envisioned. One object of the device and method is to promote both well oxygenated CPR induced blood flow, reduce ischemia related cell and organ damage, reduce blood clothing in the micro vasculature and reduce reperfusion damage.

### BRIEF DESCRIPTION OF THE DRAWINGS

Embodiments of the invention will be described, by way of example only, with reference to the drawings, in which
Fig. 1 schematically illustrates a device according to the present invention,
Fig. 2 is a schematic view of a thorax,
Fig. 3 is a schematic side view of a CPR pad,
Fig. 4 is a schematic bottom view of CPR pad,
Fig. 5 is a schematic view of an embodiment of a device including stimulation devices,
Figs. 6 and 7 schematically illustrate embodiments of a stimulation device, and
Fig. 8 schematically illustrates steps of a method according to the present invention.

### DETAILED DESCRIPTION OF EMBODIMENTS

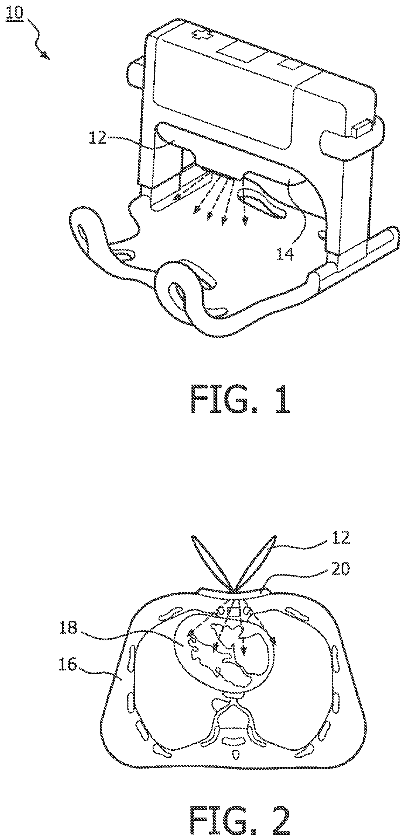

Fig. 1 schematically illustrates an embodiment of an automated device 10 where a transducer 12 is integrated in a CPR pad 14. In this way the heart and large vessels in the thorax of a patient being treated with the device 10 will be exposed to sound and/or vibrations in a physiologic important frequency / power range. Also an acoustic coupling medium may be introduced in between the transducer 12 (for example Aquaflex Ultrasonic gel pad) and the patient. The gel-pad, not illustrated here, also functions as a flexible contact surface between the CPR pad 14 and the chest of the victim/patient. The transducer may either emit, which is presently preferred, in the frequency range below 10 MHz (e.g. at 24-27 kHz, around 300 Hz) or at other suitable frequency or frequency interval. The (ultra) sound transducer may advantageously be of a Piezo type. The emitted power may advantageously be below 1W/cm2. As mentioned above the stimulation device may emit power such that tissue damage does not occur. The emitted power level should be such that local body heating is limited to below 1 degree Celsius and that clinical beneficial effects are significant. Typical power densities are around 1W/cm2 for ultra sound and acoustic sound waves but upper level values of 2-3W/cm2 may be used in some cases. The useable power density depends on the absorption coefficient of the radiation/vibration energy. It is contemplated to be advantageous to emit signals at a relatively low power level that still shows clinical benefit.

The sound or ultra-sound may either be emitted continuous but may advantageously be in a pulsed mode with a certain duty cycle. The pulsation frequency can be in a wide range from 1Hz up to a 10's of kHz, preferably in the range of 50 Hz to 500Hz. Duty cycles can be between 1% and 90% preferably less than 50%.

Instead of sound also vibration energy with a frequency higher than 1 Hz and lower than 10 kHz may be used, frequencies around 300 Hz is advantageous. The actuator in this case may be a small DC motor with an unbalanced load, there are other implementations possible. The frequency may be between 2Hz and a few thousand hertz, preferably around 300Hz.

Fig 2 is a schematic drawing of cross section of the thorax 16 with indication of heart 18 and large vessels and sound/vibration transducer. The CPR pad 12 is in contact with the thorax 16 via a gel-pad 20. Via the CPR-pad 12 the transducer emits sound waves towards the heart 18 as indicated by the arrows.

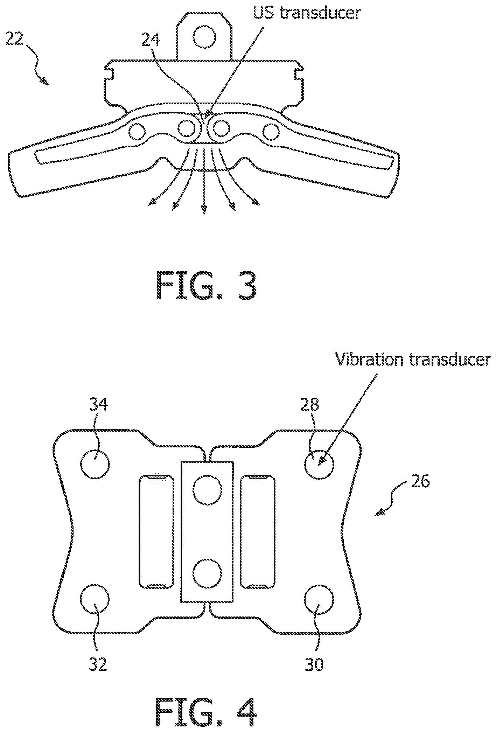

Fig. 3 is a schematic side view of a CPR pad 22 and separate sound or vibration transducer 24 in central element. The transducer 24 emit sound, preferably ultra sound, as indicated by the arrows, the specific path of the sound depends on the conditions in the thorax of the patient under treatment.

Fig. 4 is a schematic bottom view of CPR pad 26 with vibration transducers, 28, 30, 32 and 34 spread out over three parts of the pad 26.

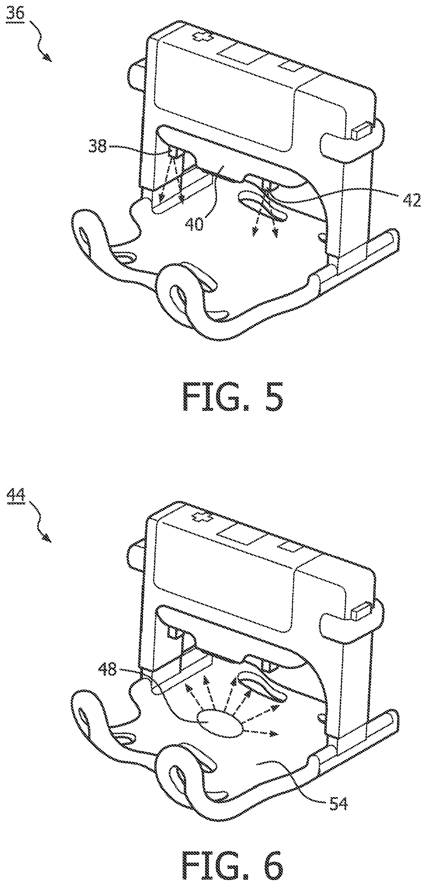

Fig. 5 is a schematic view of an embodiment of a device 36 with stimulation devices, 38 and 40, i.e. sound/vibration transducers mounted adjacent to the CPR pad 42 and fixed in the compression unit. Here the sound, ultra-sound or vibration transducer, or possibly transducers 38,40, are integrated in the compression unit adjacent to the CPR pad. Stimulation devices 38,40 can be brought in contact with the chest automatically or by a simple spring loaded system. Energies, frequencies etc. may be similar to the other embodiments described herein.

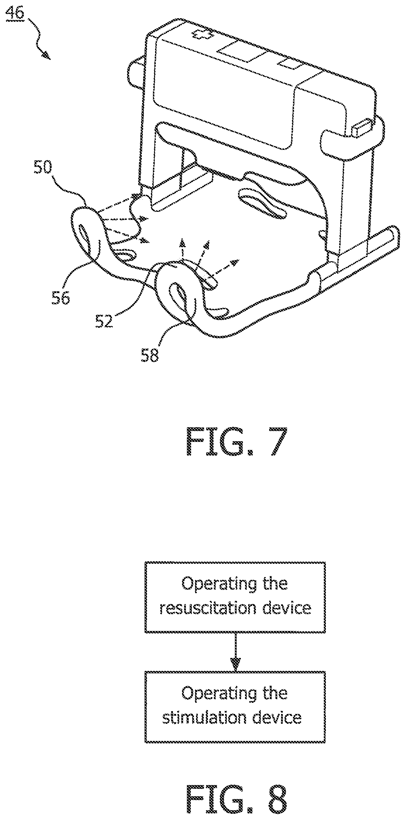

Fig. 6 and Fig. 7 schematically illustrates embodiments of the stimulation device 44 and 46, where transducer 48, 50,52 is mounted either in the bottom plate 54 of the back board or in the shoulder stops 56, 58. The location in the shoulder stop 56,58 has advantages with respect to patient contact and location of contact area, clothes are less an issue in this area.

Fig. 8 schematically illustrates steps of a method 60 according to the present invention. The method 60 is a method of operating an apparatus, such as that described in relation to the above figures. The apparatus is an apparatus for performing cardiopulmonary resuscitation comprising a resuscitation device configured for autonomously performing cardiopulmonary resuscitation, a stimulation device configured for stimulating the nitric oxide synthase pathway in a patient in need thereof and a controller for controlling the resuscitation device and/or the stimulation device. The method 60 comprises the steps of operating 62 the resuscitation device for delivering suitable resuscitation operations to a patient in need thereof, and operating 64 the stimulation device so as to stimulate the endothelial function in a patient in need thereof. The method may be implemented as a computer program to be run on the controller for controlling the resuscitation device and/or the stimulation device.

The invention is defined by the claims.

## Claims

1. An apparatus for performing cardiopulmonary resuscitation comprising:
a resuscitation device configured for autonomously performing cardiopulmonary resuscitation,
a stimulation device configured for targeted stimulation of the endothelial function including a nitric oxide synthase pathway in a patient in need thereof, and
a controller for controlling the resuscitation device and the stimulation device during cardiopulmonary resuscitation,
wherein the stimulation device is configured for stimulating the endothelial function by transmitting, when the stimulation device is in contact with the body of the patient, vibrations to target areas of the patient where nitric oxide synthase pathways are present,
wherein the stimulation device comprises one or more transducers configured for transmitting vibrations by emitting acoustic sound waves and/or ultrasound waves within a frequency range of 1 Hz to 10 MHz, and
wherein the stimulation device is further configured for altering the amplitude of the stimulation to compensate for damping of vibrations through the body of the patient.

2. The apparatus according to claim 1 wherein the stimulation device is further configured to emit acoustic sound waves and/or ultrasound waves as vibrations having a frequency in the interval from 24-27 kHz.

3. The apparatus according to claim 1 wherein the stimulation device is further configured to emit blue light as energetic electromagnetic radiation.

4. The apparatus according to claim 1 wherein the stimulation device is further configured to emit pulsed electric or magnetic fields or pulsed energetic electromagnetic radiation.

5. The apparatus according to any one of the claims 1-4, wherein the stimulation device is further configured to emit power below 10 W/cm2, such as below 8 W/cm2, such as below 5 W/cm2, such as below 3 W/cm2, such as below 2, W/cm2, such as below 1 W/cm2.

6. The apparatus according to any one of the claims 1-5, wherein the stimulation device is further configured to emit continuous or in a pulsed mode with a specific duty cycle.

7. The apparatus according to claim 6, wherein the duty cycle is between 1% and 90%, such as less than 50%.

8. The apparatus according to any one of the claims 1-7, wherein the stimulation device is configured to stimulate the thorax and/or neck/head and/or abdomen region of a patient in need thereof.

9. The apparatus according to claim 1, wherein the stimulation device is configured to emit vibrations having a vibration energy with a frequency higher than 1 Hz and lower than 10 kHz.

## Patentansprüche

1. Gerät zur Durchführung einer kardiopulmonalen Wiederbelebung, die Folgendes umfasst: eine Wiederbelebungsgerät, die so konfiguriert ist, dass sie autonom Folgendes durchführt:
kardiopulmonale Reanimation,
ein Stimulationsgerät, die zur gezielten Stimulation der Endothelfunktion einschließlich eines Stickoxid-Synthase-Wegs bei einem Patienten, der dies benötigt,
konfiguriert ist, und
einen Controller zur Steuerung des Wiederbelebungsgeräts und des Stimulationsgeräts während der Herz-Lungen-Wiederbelebung,
wobei das Stimulationsgerät zum Stimulieren der Endothelfunktion durch Übertragung von Vibrationen auf Zielbereiche des Patienten, in denen Stickoxid-Synthase-Wege vorhanden sind, konfiguriert ist, wenn die Stimulationsgerät in Kontakt mit dem Körper des Patienten ist,
wobei die Stimulationsgerät einen oder mehrere Wandler umfasst, die zum Übertragen von Vibrationen durch Aussenden von akustischen Schallwellen und/oder Ultraschallwellen innerhalb eines Frequenzbereichs von 1 Hz bis 10 MHz konfiguriert sind, und
wobei das Stimulationsgerät ferner so konfiguriert ist, dass es die Amplitude der Stimulation ändert, um die Dämpfung von Schwingungen durch den Körper des Patienten zu kompensieren.

2. Gerät nach Anspruch 1, wobei das Stimulationsgerät ferner so konfiguriert ist, dass es akustische Schallwellen und/oder Ultraschallwellen als Vibrationen mit einer Frequenz im Bereich von 24-27 kHz aussendet.

3. Gerät nach Anspruch 1, wobei das Stimulationsgerät ferner so konfiguriert ist, dass es blaues Licht als energetische elektromagnetische Strahlung emittiert.

4. Gerät nach Anspruch 1, wobei das Stimulationsgerät ferner so konfiguriert ist, dass es gepulste elektrische oder magnetische Felder oder gepulste energetische elektromagnetische Strahlung emittiert.

5. Gerät nach einem oder mehreren der Ansprüche 1 bis 4 entspricht, wobei das Stimulationsgerät weiterhin so konfiguriert ist, dass es eine Leistung von weniger als 10 W/cm2, z. B. weniger als 8 W/cm2, z. B. weniger als 5 W/cm2, z.B. weniger als 3 W/cm2, z. B. weniger als 2 W/cm2, z. B. weniger als 1 W/cm2, abgibt.

6. Gerät nach einem der Ansprüche 1 bis 5, wobei das Stimulationsgerät ferner so konfiguriert ist, dass es kontinuierlich oder in einem gepulsten Modus mit einem spezifischen Arbeitszyklus emittiert.

7. Gerät nach Anspruch 6, wobei der Arbeitszyklus zwischen 1 % und 90 % liegt, z. B. weniger als 50 %.

8. Gerät nach einem der Ansprüche 1 bis 7, wobei das Stimulationsgerät so konfiguriert ist, dass es den Thorax und/oder den Hals/Kopf und/oder die Bauchregion eines Patienten, der dies benötigt, stimuliert.

9. Gerät nach Anspruch 1, wobei das Stimulationsgerät so konfiguriert ist, dass es Vibrationen mit einer Vibrationsenergie mit einer Frequenz von mehr als 1 Hz und weniger als 10 kHz aussendet.

## Revendications

1. Appareil permettant d'effectuer une réanimation cardiopulmonaire comprenant :
un dispositif de réanimation conçu pour effectuer de manière autonome une réanimation cardiopulmonaire,
un dispositif de stimulation conçu pour stimuler de manière ciblée la fonction endothéliale comprenant une voie de l'oxyde nitrique synthase chez un patient ayant besoin d'une telle stimulation, et
un dispositif de commande pour commander le dispositif de réanimation et le dispositif de stimulation lors de la réanimation cardiopulmonaire,
dans lequel le dispositif de stimulation est conçu pour stimuler la fonction endothéliale par transmission, lorsque le dispositif de stimulation est en contact avec le corps du patient, des vibrations vers des zones cibles du patient où des voies de l'oxyde nitrique synthase sont présentes,
dans lequel le dispositif de stimulation comprend une pluralité de transducteurs conçus pour transmettre des vibrations par émission des ondes sonores acoustiques et/ou des ondes ultrasonores dans une plage de fréquences comprise entre 1 Hz et 10 MHz, et
dans lequel le dispositif de stimulation est en outre conçu pour modifier l'amplitude de la stimulation afin de compenser l'amortissement des vibrations à travers le corps du patient.

2. Appareil selon la revendication 1, dans lequel le dispositif de stimulation est en outre conçu pour émettre des ondes sonores acoustiques et/ou des ondes ultrasonores sous forme de vibrations présentant une fréquence dans l'intervalle compris entre 24 et 27 kHz.

3. Appareil selon la revendication 1, dans lequel le dispositif de stimulation est en outre conçu pour émettre la lumière bleue sous forme de rayonnement électromagnétique énergétique.

4. Appareil selon la revendication 1, dans lequel le dispositif de stimulation est en outre conçu pour émettre des champs électriques ou magnétiques pulsés ou un rayonnement électromagnétique énergétique pulsé .

5. Appareil selon l'une quelconque des revendications 1 à 4, dans lequel le dispositif de stimulation est en outre conçu pour émettre une puissance inférieure à 10 W/cm2, telle qu'inférieure à 8 W/cm2, telle qu'inférieure à 5 W/cm2, telle qu'inférieure à 3 W/cm2, telle qu'inférieure à 2 W/cm2, telle qu'inférieure à 1 W/cm2.

6. Appareil selon l'une quelconque des revendications 1 à 5, dans lequel le dispositif de stimulation est en outre conçu pour émettre en continu ou en mode pulsé à un rapport cyclique spécifique.

7. Appareil selon la revendication 6, dans lequel le rapport cyclique est dans la plage comprise entre 1 % et 90 %, tel qu'inférieur à 50 %.

8. Appareil selon l'une quelconque des revendications 1 à 7, dans lequel le dispositif de stimulation est conçu pour stimuler le thorax et/ou le cou/la tête et/ou la zone abdominale d'un patient ayant besoin d'une telle stimulation.

9. Appareil selon la revendication 1, dans lequel le dispositif de stimulation est conçu pour émettre des vibrations présentant une énergie de vibration à une fréquence supérieure à 1 Hz et inférieure à 10 kHz.
